# EUROPEAN PATENT SPECIFICATION

(11) **EP 3 883 618 B1**
(45) Date of publication and mention of the grant of the patent: **02.08.2023**
(21) Application number: 19824388.3
(22) Date of filing: 18.11.2019
(51) Int. Cl.: A61L 27/20, A61L 27/50, A61L 27/52

(54) **INJECTABLE COMPOSITION INCLUDING HYALURONIC ACID AND USE OF SAID COMPOSITION**
INJIZIERBARE ZUSAMMENSETZUNG MIT HYALURONSÄURE UND VERWENDUNG DER BESAGTEN ZUSAMMENSETZUNG
COMPOSITION INJECTABLE COMPRENANT DE L'ACIDE HYALURONIQUE ET UTILISATION DE LADITE COMPOSITION

(30) Priority: 19.11.2018 IT 201800010415
(43) Date of publication of application: 29.09.2021
(73) Proprietor: Innate S.r.l., 15067 Novi Ligure (AL) (IT)
(72) Inventor: PANZIERI, Federico, 15067 Novi Ligure (AL) (IT)
(74) Representative: Bottino, Giovanni
(86) International application number: PCT/IB2019/059887
(87) International publication number: WO 2020/104915

(56) References cited:
- WO-A1-2012/062775
- US-B2- 8 623 839

## Description

The present invention relates to an injectable composition in the form of hydrogel, comprising water and hyaluronic acid.

The hyaluronic acid is a very biocompatible natural polysaccharide, present in all human tissues, and is one of the fundamental components of connective tissues. It is distributed ubiquitously in animal tissues and fluids, in high concentrations in synovial fluids, in vitreous humour and in the skin, and it is the main responsible for the viscosity and lubricating activity of synovial liquid. In cartilage, the hyaluronic acid acts as a support for the aggregation of proteoglycans and proteins.

The hyaluronic acid is a long glycosaminoglycan composed of repeated disaccharides of glucuronic acid and N-acetylglucosamine with high molecular weight and high viscosity.

The hyaluronic acid is currently prepared in gel form from a powder in which it is present in the form of salt (sodium hyaluronate), and in such gel it forms water micelles thanks to its high affinity with the water itself. A crystalline gel is then formed which, injected, obtains a prolonged duration in the tissues, since it maintains biocompatibility.

The hyaluronic acid is used e.g. in aesthetic medicine to increase the volume of facial tissues, to correct wrinkles, skin folds, to increase the volume of the lips, and in general for the correction of skin blemishes. In this case it can be injected into a scar or at the level of the superficial dermal layer for skin treatments and act as a moisturiser for the dermis or even as a filler and therefore anti-wrinkle. This action is made possible thanks to the viscoelastic and moisturising properties of the hyaluronic acid, which is naturally present in the extracellular matrix of the skin with the function of regulating hydration and elasticity. The intradermal administration of the device gives the tissue a good amount of hyaluronic acid to counteract the process of skin ageing.

Alternatively, the hyaluronic acid is used for intra-articular administration, in the treatment of osteoarthritis, e.g. at the knee level, in which it is found naturally in the synovial liquid, of which it is the main responsible of the high lubricating capacity thanks to its viscosity. The hyaluronic acid helps to lubricate the joint and cushion mechanical stress, and therefore has a lubricating function and a damping function at the same time. Furthermore, it protects the cartilage from the penetration of inflammatory cells and from the lytic enzymes which degrade it.

In intra-articular infiltrations, the preparation generally consists of a sodium salt of a high molecular weight hyaluronic acid fraction with a high degree of purity. The short half-life of this molecule at the joint level, excludes that its effectiveness is linked simply to the restoration of the physiological levels of hyaluronic acid in the osteoarthrosic joints. The evaluations performed in patients with arthrosis and other arthropathies with knee involvement have shown an action that, in principle, exerts an anti-inflammatory activity of the molecule.

Microartroscopy studies and ultrastructural studies on joint cartilage and synovial membrane have also shown that in humans there is a possible repair of degenerative joint injuries following the intra-articular use of the drug.

Both at skin level and at an intra-articular level the compositions in the form of hydrogels with hyaluronic acid currently known clash with the technical problem of ensuring a good viscosupplementation to the joint or to the skin and at the same time allow an easy extrusion from the needle of the injection syringe. The high viscosities of the hydrogel, in fact, guarantee good viscosupplementations but can create problems during extrusion, since the injection needles typically have very small sections. Therefore, if the hydrogel is too viscous, it may be necessary to give too much force during the injection, generating a greater risk both of creating microtrauma for the patient and possibly breaking the vial.

At the same time, compositions of this type have to deal with the degradation of hyaluronic acid over time; such degradation in fact affects the so-called shelf life, i.e. the duration of the period from production to sale in which it is necessary to keep the total quality of the product intact, as well as the duration of the effect on the body following administration. The duration of the hyaluronic acid strongly depends on the type of treatment to which it is subjected and its composition. Two categories can be distinguished: linear hyaluronic acid and cross-linked hyaluronic acid. In order to pass from linear hyaluronic acid to cross-linked one, specific cross-linking agents are used, such as BDDE (1,4-butanediol diglidyl ether) or DVS (Divinyl Sulfone).

Currently the compositions which exhibit a greater duration are those which comprise cross-linked hyaluronic acid. However, the presence of traces of BDDE or other cross-linking agents at the end of the cross-linking reaction is problematic due to various aspects, e.g. because it can immediately trigger actions of intracutaneous reactivity and in the long term can cause the onset of under-skin granulomas.

A main cause of the degradation of hyaluronic acid is the action of the hyaluronidase enzyme. Such enzyme acts very effectively on linear hyaluronic acid, while the three-dimensional structure assumed by the cross-linked hyaluronic acid thanks to the cross-linking process makes the enzyme action difficult.

Therefore, according to the state of the art, the request for a longer duration of the composition requires the use of cross-linked hyaluronic acid, but at the expense of an increase in risks for the health and safety of the user.

Document WO2012/062775 describes a composition comprising hyaluronic acid, a cross-linked part of which consists of a mixture of low molecular weight hyaluronic acid and a greater molecular weight hyaluronic acid for an improved ease of extrusion through the injection needle. The document indicates that for the linear or only slightly cross-linked hyaluronic acid such expedient is not necessary.

However, experimental tests have shown that even the linear hyaluronic acid can have similar problems in guaranteeing, at the same time, a good viscosupplementation and an ease of extrusion through the injection needle.

Furthermore, in order to guarantee a longer duration of the hyaluronic acid, the use of trehalose is known, a disaccharide composed of two molecules of glucose, known to be a strong antioxidant and a stabilizing agent, which acts as a protector of biomolecules against environmental stresses, e.g., high temperatures. The use of trehalose as a stabilizer in a composition comprising hyaluronic acid is described, e.g., in document US8623839.

Currently, there is therefore an unmet need for a composition in the form of a hydrogel comprising hyaluronic acid which, at the same time, guarantees a good viscosupplementation and an ease of extrusion through the injection needle, as well as a longer duration and lower risks for the patient.

The present invention meets this technical requirement and overcomes the above limitations relating to the compositions currently known of the state of the art and aims at constituting a functional and advantageous solution both in terms of construction and use.

These objects are obtained, according to the invention, by providing an injectable composition in the form of a hydrogel, comprising water, hyaluronic acid and trehalose for inhibiting the action of hyaluronidase in the injected condition of the composition, wherein the said hyaluronic acid is of the linear type and comprises a medium molecular weight sodium hyaluronate between 800 KDa and 2000 KDa, preferably between 1200 KDa and 1500 KDa, and low molecular weight sodium hyaluronate less than 700 KDa, preferably between 200 and 400 KDa.

The mixture of linear hyaluronic acid with medium molecular weight and low molecular weight allows a high concentration of hyaluronic acid in the composition, for an increased viscosupplementation, while ensuring a facilitated extrusion for the hydrogel. This proved to be unexpectedly advantageous also for linear hyaluronic acid with respect to what indicated by the state of the art, wherein this aspect is described only in relation to cross-linked hyaluronic acid.

In this way it is possible to effectively use linear hyaluronic acid, which does not present the problems for the patient's health exhibited instead by cross-linked hyaluronic acid.

In this perspective, the presence in the composition of trehalose becomes crucial, to counterbalance the shorter duration of the linear hyaluronic acid compared to the cross-linked one. If trehalose is indeed a known protector of hyaluronic acid from environmental stresses such as heat, its inhibitory effect on hyaluronidase is not currently described and has been recognized only thanks to specific tests on hyaluronidase, performed after the analysis of the results of biocompatibility studies, which revealed the presence of the hyaluronic acid molecule even after eight weeks.

Cross-linked hyaluronic acid has an altered three-dimensional shape which makes it not very susceptible to hyaluronidase action, whereas this does not happen for linear hyaluronic acid. The present invention therefore arises from the surprising observation that linear hyaluronic acid can be protected from trehalose by masking the sites of hyaluronidase action on the hyaluronic acid by the trehalose itself.

Moreover, the trehalose does not generate toxicity or intracutaneous problems, which are specific to cross-linked hyaluronic acid, and are in fact excreted as such or split into two glucose molecules if the patient has trehalase, which is not present in the whole population.

The composition object of the present invention is therefore a valid and safer substitute for the currently known long-life products based on cross-linked hyaluronic acid.

According to an exemplary embodiment, the hyaluronic acid is provided in a percentage by weight comprised between 1.6% and 3%.

According to an improvement, the said hyaluronic acid consists of the said medium molecular weight sodium hyaluronate in a percentage by weight comprised between 70% and 90% and the said low molecular weight sodium hyaluronate in a percentage by weight comprised between 10% and 30%.

In a further exemplary embodiment, the trehalose is provided in a percentage by weight comprised between 0.5% and 5%.

In an exemplary embodiment the viscosity of the composition is at least 70000 cP. This value is suitable both for intra-articular viscosupplementation and for intradermal use.

In the composition the trehalose is not only a functional excipient able to protect hyaluronic acid, slowing down its degradation, making hyaluronic acid less attackable from external agents, e.g. from heat, but acts against the hyaluronidase action allowing it to maintain the viscosupplementation characteristics over time. Experimental tests have shown that in a composition without trehalose, the hyaluronic acid is degraded by a standard 50% hyaluronidase after one hour; on the contrary, with the presence of trehalose in the percentages described above, the hyaluronic acid remains unchanged after one hour.

Thanks to this protection of the hyaluronic acid over time, the finished product thus acquires greater stability and greater durability over time.

Experimental tests have also highlighted that, thanks to the trehalose action, after almost 2 years, the viscosity of the hydrogel decreases only by about 5%, in particular from 84231 cP to 80123 cP in the analysed compositions.

The trehalose also acts to protect the hyaluronic acid during sterilization and, once the product is administered, exerts an important anti-inflammatory activity in the patient.

The composition can also advantageously comprise excipients to ensure stability and functionality.

In an embodiment, sodium chloride is included. This allows the adjustment of the osmolarity of the composition.

In a further embodiment, a buffer system is included. Such a buffer system may be of any suitable type.

Preferably the buffer system comprises dibasic sodium phosphate and monobasic sodium phosphate.

The buffer system can alternatively be composed of other salts, e.g. the equivalent potassium salts, i.e. dibasic potassium phosphate and monobasic potassium phosphate.

In a further variant, the buffer system can be a citrate buffer, comprising citric acid and sodium citrate.

The buffer can be provided in all its forms of hydration.

According to an exemplary example, the said dibasic sodium phosphate is in anhydrous and/or dihydrate and/or dodecahydrate hydration state and the said monobasic sodium phosphate is in anhydrous and/or dihydrate hydration state.

Similarly, such hydration states can be envisaged in the above mentioned cases of a buffer system comprising potassium salts or a citrate buffer system.

It is also possible to provide amino acids in the composition.

In this case, the trehalose also acts to protect the amino acids possibly added to the product.

Advantageously, the composition has the appearance of a transparent gel, an important condition for the visual control of the syringes.

The composition described above for use in an intra-articular injection to increase the lubricating capacity of the synovial liquid of the joint is also an object of the present invention.

The use of the composition described above in an intradermal injection to correct the skin blemishes is also an object of the present invention. The composition has a moisturising effect on the skin and gives firmness, since it acts inside the layers of the dermis, increasing the volume and obtaining a corrective effect of the skin imperfections.

The composition described above for use in an PRP administration is also an object of the present invention.

The platelet-rich plasma is a known technique used to stimulate and accelerate the healing of bones and soft tissues. Such technique uses an autologous plasma volume which has a platelet concentration above the base value. The normal number of platelets in the blood varies between 150,000/µl and 350,000/µl, with an average of about 200,000/µl. The concentration useful for the regenerative effect on bones and soft tissues is generally recognized in 1,000,000/µl of platelets, typically used in a volume of 5 ml of plasma. The PRP injection generally takes place with hyaluronic acid, which exerts a viscosupplementation necessary for the success of the administration. The composition object of the present invention has proved to be particularly advantageous in this type of application.

These and other features of the invention and the advantages deriving therefrom will become apparent from the following detailed description of an embodiment, which is preferred among the advantageous and various embodiments of the invention, illustrated by way of a mere not limiting example, with refer to the table below.

| **Component** | **Percentage by weight** |
|---|---|
| Water for injectable solutions | 95.620 |
| Trehalose | 1.000 |
| Sodium hyaluronate | 2.500 |
| Sodium chloride | 0.700 |
| Dibasic sodium phosphate | 0.150 |
| Monobasic sodium phosphate | 0.030 |

In this embodiment, the sodium hyaluronate consists of 80% by weight of medium molecular weight sodium hyaluronate and 20% by weight of low molecular weight sodium hyaluronate.

With the weight percentage values indicated in the table, the total viscosity of the hydrogel remains very satisfactory as regards the viscosupplementation, but it is reduced to the advantage of the extrusion from the injection needle. This occurs because the presence of low molecular weight hyaluronic acid offers a reduced contribution to the final viscosity.

The above composition has the following physical, microbiological and chemical characteristics:
- pH: 6.50 - 7.50
- osmolality: 270 - 400 mOsm/Kg
- density: 1,000 - 1,015 g/cm³
- appearance: transparent gel
- viscosity: at least 70000 cP
- titer: maximum concentration of 25 mg/ml (this concentration must be suitable for both uses, intradermal and intra-articular use).
- Sterile
- Endotoxins <5 EU / mL

## Claims

1. Injectable composition in the form of hydrogel, comprising water and
hyaluronic acid,
**characterised in that**
it comprises trehalose for inhibiting the hyaluronidase action in the injected condition of the composition, and the said hyaluronic acid is of the linear type and comprises medium molecular weight sodium hyaluronate between 800 KDa and 2000 KDa, preferably between 1200 KDa and 1500 KDa, and low molecular weight sodium hyaluronate less than 700 KDa, preferably between 200 and 400 KDa.

2. Composition according to claim 1, wherein the hyaluronic acid is provided in a percentage by weight comprised between 1.6% and 3%.

3. Composition according to claim 1 or 2, wherein the trehalose is provided in a percentage by weight comprised between 0.5% and 5%.

4. Composition according to one or more of the preceding claims wherein the said hyaluronic acid consists of the said medium molecular weight sodium hyaluronate in a percentage by weight comprised between 70% and 90% and the said low molecular weight sodium hyaluronate in a percentage by weight comprised between 10% and 30%.

5. Composition according to one or more of the preceding claims, wherein sodium chloride is included.

6. Composition according to one or more of the preceding claims, wherein a buffer system comprising dibasic sodium phosphate and monobasic sodium phosphate is included.

7. Composition according to claim 6, wherein the said dibasic sodium phosphate is in anhydrous and/or dihydrate and/or dodecahydrate hydration state and the said monobasic sodium phosphate is in anhydrous and/or dihydrate hydration state.

8. Composition according to one or more of claims 1 to 7 for use in an intra-articular injection in a joint to increase the lubricating capacity of the synovial liquid of the joint.

9. Use of the composition according to one or more of claims 1 to 7 in an intradermal injection for the correction of skin blemishes.

10. Composition according to one or more of claims 1 to 7 for use in an PRP administration.

## Patentansprüche

1. Injizierbare Zusammensetzung in Form eines Hydrogels, umfassend Wasser und Hyaluronsäure,
**dadurch gekennzeichnet, dass**
sie Trehalose zum Hemmen der Hyaluronidasewirkung in dem injizierten Zustand der Zusammensetzung umfasst und die Hyaluronsäure vom linearen Typ ist und Natriumhyaluronat mit mittlerem Molekulargewicht zwischen 800 KDa und 2000 KDa, bevorzugt zwischen 1200 KDa und 1500 KDa, und Natriumhyaluronat mit niedrigem Molekulargewicht von weniger als 700 KDa, bevorzugt zwischen 200 und 400 KDa, umfasst.

2. Zusammensetzung nach Anspruch 1, wobei die Hyaluronsäure in einem Gewichtsprozentsatz zwischen 1,6 % und 3 % bereitgestellt ist.

3. Zusammensetzung nach Anspruch 1 oder 2, wobei die Trehalose in einem Gewichtsprozentsatz zwischen 0,5 % und 5 % bereitgestellt ist.

4. Zusammensetzung nach einem oder mehreren der vorstehenden Ansprüche, wobei die Hyaluronsäure aus dem Natriumhyaluronat mit mittlerem Molekulargewicht in einem Gewichtsprozentsatz zwischen 70 % und 90 % und dem Natriumhyaluronat mit niedrigem Molekulargewicht in einem Gewichtsprozentsatz zwischen 10 % und 30 % besteht.

5. Zusammensetzung nach einem oder mehreren der vorstehenden Ansprüche, wobei Natriumchlorid enthalten ist.

6. Zusammensetzung nach einem oder mehreren der vorstehenden Ansprüche, wobei ein Puffersystem, das zweibasiges Natriumphosphat und einbasiges Natriumphosphat umfasst, enthalten ist.

7. Zusammensetzung nach Anspruch 6, wobei das zweibasige Natriumphosphat in einem wasserfreien und/oder Dihydrat- und/oder Dodekahydrat-Hydratationszustand vorliegt und das einbasige Natriumphosphat in einem wasserfreien und/oder Dihydrat-Hydratationszustand vorliegt.

8. Zusammensetzung nach einem oder mehreren der Ansprüche 1 bis 7 zur Verwendung bei einer intraartikulären Injektion in ein Gelenk, um die Schmierkapazität der Synovialflüssigkeit des Gelenks zu erhöhen.

9. Verwendung der Zusammensetzung nach einem oder mehreren der Ansprüche 1 bis 7 in einer intradermalen Injektion zur Korrektur von Hautunreinheiten.

10. Zusammensetzung nach einem oder mehreren der Ansprüche 1 bis 7 zur Verwendung in einer PRP-Verabreichung.

## Revendications

1. Composition injectable sous la forme d'un hydrogel, comprenant de l'eau et de l'acide hyaluronique,
**caractérisée en ce qu'**
elle comprend du tréhalose pour inhiber l'action de la hyaluronidase dans l'état injecté de la composition, et ledit acide hyaluronique est de type linéaire et comprend du hyaluronate de sodium de poids moléculaire moyen situé entre 800 kDa et 2000 kDa, de préférence entre 1200 kDa et 1500 kDa, et du hyaluronate de sodium de poids faible moléculaire inférieur à 700 kDa, de préférence entre 200 et 400 kDa.

2. Composition selon la revendication 1, dans laquelle l'acide hyaluronique est fourni en un pourcentage en poids compris entre 1,6 % et 3 %.

3. Composition selon la revendication 1 ou 2, dans laquelle le tréhalose est fourni en un pourcentage en poids compris entre 0,5 % et 5 %.

4. Composition selon l'une ou plusieurs des revendications précédentes, dans laquelle ledit acide hyaluronique est constitué dudit hyaluronate de sodium de poids moléculaire moyen en un pourcentage en poids compris entre 70 % et 90 % et dudit hyaluronate de sodium de faible poids moléculaire en un pourcentage en poids compris entre 10 % et 30 %.

5. Composition selon l'une ou plusieurs des revendications précédentes, dans laquelle du chlorure de sodium est inclus.

6. Composition selon l'une ou plusieurs des revendications précédentes, dans laquelle un système tampon comprenant du phosphate de sodium dibasique et du phosphate de sodium monobasique est inclus.

7. Composition selon la revendication 6, dans laquelle ledit phosphate de sodium dibasique est dans un état d'hydratation anhydre et/ou dihydraté et/ou dodécahydraté et ledit phosphate de sodium monobasique est un état d'hydratation anhydre et/ou dihydraté.

8. Composition selon l'une ou plusieurs des revendications 1 à 7, pour une utilisation dans une injection intra-articulaire dans une articulation pour augmenter la capacité de lubrification du liquide synovial de l'articulation.

9. Utilisation de la composition selon l'une ou plusieurs des revendications 1 à 7 dans une injection intradermique pour la correction des imperfections de la peau.

10. Composition selon l'une ou plusieurs des revendications 1 à 7, pour une utilisation dans une administration de PRP.
